# EUROPÄISCHE PATENTSCHRIFT

(11) **EP 0 912 140 B1**
(45) Veröffentlichungstag und Bekanntmachung des Hinweises auf die Patenterteilung: **25.08.2004**
(21) Anmeldenummer: 97931646.0
(22) Anmeldetag: 24.06.1997
(51) Int. Cl.: A61B 17/28

(54) **ABWINKELBARES ENDOSKOPISCHES INSTRUMENT**
ENDOSCOPIC INSTRUMENT WHICH CAN BE BENT
INSTRUMENT ENDOSCOPIQUE POUVANT ETRE PLIE

(30) Priorität: 24.06.1996 DE 19625241
(43) Veröffentlichungstag der Anmeldung: 06.05.1999
(73) Patentinhaber: Karl Storz GmbH & Co. KG, 78532 Tuttlingen (DE)
(72) Erfinder: LAFOND, Christophe, F-03200 Vichy (FR); TANGUY, Alain, F-63000 Clermont-Ferrand (FR); BACHER, Uwe, D-78532 Tuttlingen (DE)
(74) Vertreter: Lohr, Georg, Dr.
(86) Internationale Anmeldenummer: PCT/DE1997/001309
(87) Internationale Veröffentlichungsnummer: WO 1997/049342

(56) Entgegenhaltungen:
- EP-A- 0 606 531
- EP-A- 0 609 503
- DE-U- 9 418 094
- US-A- 5 330 502
- US-A- 5 456 684

## Beschreibung

### Technisches Gebiet

Die Erfindung bezieht sich auf ein abwinkelbares endoskopisches Instrument gemäß dem Oberbegriff des Patentanspruchs 1.

### Stand der Technik

Endoskopische Instrumente, deren distales Ende als Einheit gegenüber der Längsachse des Instruments abwinkelbar bzw. schwenkbar ist, sind bekannt. Beispielhaft wird auf folgende Druchschriften verwiesen: US-PS 5,318,528, US-PS 5,330,502, US-PS 5,354,311, DE 42 43 715 A1, DE 43 24 254 C1.

Dabei kann an dem distalen Ende ein beliebiges Funktionselement angebracht sein. Dieses Funktionselement ist über eine Stange mit einem proximal angeordneten Betätigungselement derart verbunden, daß die Winkelstellung des Funktionselements, also beispielsweise eines Zangen- oder Scherenmauls relativ zu dem als Einheit abwinkelbaren bzw. schwenkbaren distalen Ende eingestellt werden kann.

Die aus den vorgenannten Druckschriften bekannten Instrumente haben den Nachteil, daß entweder das distale Ende nicht über einen großen Schwenkbereich abwinkelbar ist, oder die Instrumente einen vergleichsweise großen Platzbedarf haben, so daß sie beispielsweise nicht an den Wirbeln bei jungen Menschen einsetzbar sind.

Darüber hinaus sind die bekannten Instrumente nicht leicht zu sterilisieren.

### Darstellung der Erfindung

Erfindungsgemäß ist nun erkannt worden, daß man ein besonders kompaktes und gleichzeitig stabiles endoskopisches Instrument dann erhält, wenn die Schwenkachse des distalen Endes die Längsachse des Endoskops nicht schneidet. Bei einer derartigen Anordnung der Schwenkachse des distalen Endes ergibt sich jedoch das Problem, daß sich bei einer Änderung der Winkelstellung des distalen Endes relativ zur Längsachse des Instruments die Stellung des sonstigen Elements relativ zu dem als Einheit schwenkbaren distalen Ende ändert. Deshalb ist erfindungsgemäß ein Instrument gemäß Anspruch 1 vorgesehen.

Die Erfindung hat die Eigenschaft, daß die Stange in an sich bekannter Weise auf Zug oder Druck das Maul der am distalen Ende vorgesehenen Zange, Schere etc. schließt bzw. die Winkelstellung des Funktionselements aus der Ruhestellung ändert.

Weiterhin ist proximalseitig ein Stellelement vorgesehen, durch dessen Betätigung das distale Ende abwinkelbar bzw. schwenkbar ist. Dieses Stellelement kann insbesondere eine Stellschraube sein, die über einen Gewindeeingriff einen Hohlzylinder verschiebt, der konzentrisch zu der Stange angeordnet ist, und der ein Schwenkelement betätigt, daß die Winkelstellung des distalen Endes ändert und das bevorzugt fest mit dem distalen Ende verbunden ist. Das Schwenkelement und der Hohlzylinder können dabei insbesondere über einen Zapfen, der in eine Nut eingreift, miteinander in Wirkverbindung stehen.

Weiterhin dreht die Stellschraube über einen Gewindeeingriff ein Zwischenelement, das über unterschiedliche Gewinde den Hohlzylinder und zum Längenausgleich das Außenrohr geeignet verschiebt. Dabei kann das Gewinde zum Antrieb des Hohlzylinders ein Linksgewinde und das Gewinde zum Längenausgleich ein Rechtsgewinde sein. Die Stellschraube kann konzentrisch zur Längsachse oder in einem Winkel von 90° zur Längsachse angeordnet sein.

Das erfindungsgemäße abwinkelbare Instrument hat damit den Vorteil, daß das distale Ende über einen großen Schwenkbereich - typischerweise ±70 Grad und mehr gegenüber der Hauptachse verschwenkbar ist. Dennoch ist das Instrument so kompakt, daß es ohne weiteres in einen Trokar mit Standarddurchmesser einqeschoben werden kann.

Bei einer weiteren erfindungsgemäßen Ausgestaltung, ist der Hohlzylinder, der als Übertragungselement für die Schwenkbewegung des distalen Endes als Einheit dient, distalseitig in einem Bajonettelement geführt, das einen Bajonetflansch aufweist, mit dem es in eine Bajonettaufnahme in einem Außenrohr einsetzbar ist.

Hierdurch ist eine leichte Zerlegbarkeit bei hervorragender Stabilität und eine sichere Führung und vor allem ein definierter Zusammenbau des Instruments nach der Reinigung bzw. Sterilisierung im zerlegten Zustand gewährleistet.

Weiterbildungen der Erfindung sind Gegenstand der abhängigen Ansprüche.

Gemäß Anspruch 2 ist die Stange im Bereich der Abwinkelung flexibel ausgebildet. Die Stange kann dabei insbesondere einen rechteckigen Querschnitt haben, wobei die schmale Seite des Rechtecks senkrecht zur Schwenkachse angeordnet ist.

Bei der im Anspruch 3 gekennzeichneten Weiterbildung weist die Stange im Bereich der Anwinkelung einen geringeren Durchmesser als in dem nicht abgewinkelten Bereich auf, in dem sie insbesondere in üblicherweise einen runden Querschnitt haben kann.

In einer bevorzugten Weiterbildung ist das distale Ende bzw. der distale Kopf um die Längsachse in mehrere indizierte Positionen bzw. Raststellungen drehbar, so daß der Chirurg bestimmte wichtige Winkelstellungen schnell einstellen kann. Dabei ist es weiter von Vorteil, wenn ein Anschlag od. dgl. am Handgriff es möglich macht, den Kopf zur Drehung freizugeben bzw. dies in einer bestimmten Position festzustellen.

Bei einer anderen Weiterbildung weist der Hohlzylinder an seinem proximalen Endbereich zwei sich gegenüberliegende gefederte Erhöhungen auf, mit denen in komplementär ausgebildete Aufnahmen eines verdrehgesicherten Rohrs eingesetzt wird. Die Sicherung des Hohlzylinders gegen Verdrehen und damit ein Öffnen des Bajonetts erfolgt über die eingesetzte Stange, die ein Einfedern der Erhöhungen des Hohlzylinders und damit ein Verdrehen bzw. ein Zerlegen des Instruments verhindert.

Das erfindungsgemäße Instrument kann für die verschiedensten medizinischen Eingriffe eingesetzt werden. Besonders vorteilhaft ist sein Einsatz bei chirurgischen Eingriffen zur Zölioskopie bzw. Endoskopie an den Wirbeln bei jungen Menschen, bei denen die Einführung der endoskopischen Instrumente von der Vorderseite des Thorax her vorzuziehen ist, da dann Eingriffe am posterioren Abschnitt der Wirbel und zwar nacheinander an mehreren Wirbeln möglich sind, ohne daß das Instrument zurückgezogen werden müßte. Die Eingriffe können dabei dank der verschiedenen Drehbewegungen bzw. Schwenkbewegungen vom Bereich nahe des Handgriffs aus gesteuert werden.

### Kurze Beschreibung der Zeichnung

Die Erfindung wird nachstehend ohne Beschränkung des allgemeinen Erfindungsgedankens anhand von Ausführungsbeispielen unter Bezugnahme auf die Zeichnung exemplarisch beschrieben, auf die im übrigen bezüglich der Offenbarung aller im Text nicht näher erläuterten erfindungsgemäßen Einzelheiten ausdrücklich verwiesen wird. Es zeigen:
- Fig. 1a: einen Längsschnitt durch ein erfindungsgemäßes Instrument,
- Fig. 1b und 1c: vergrößert Details aus Figur 1a,
- Fig. 2a, 3 und 4: Querschnitt durch das in Figur 1a
- dargestellte: Element an den Stellen a-a, b-b und c-c, und
- Fig. 2b: eine Aufsicht auf die Schnittebene a-a.

### Darstellung eines Ausführungsbeispiels

Das in den Figuren dargestellte endoskopische Instrument weist ein distales Ende 1 auf, das als Einheit um eine Schwenkachse 1a schwenkbar ist. Die Schwenkachse 1a schneidet nicht die Längsachse 2 des Instruments, sondern weist von dieser in Richtung senkrecht zur Längsachse einen Abstand auf. Die Schwenkachse 1a ist an einem Bajonettelement 3 angebracht, das einen Bajonettflansch 31 aufweist, mit dem es in eine Bajonettaufnahme 32 eingesetzt werden kann, die in einem Außenrohr 4 des Instruments vorgesehen ist. In dem Bajonettelement 3 ist distalseitig ein Hohlzylinder 5 geführt, der ein Schwenkelement 11, in dem eine Nut 12 vorgesehen ist, mittels eines Zapfens 13 betätigt. Durch die Betätigung des Schwenkelements 11 wird die Winkelstellung des distalen Endes 1 relativ zur Längsachse 2 geändert. In dem Hohlzylinder 5 ist eine Stange 6 geführt, die mit einem nicht dargestellten, proximal angeordneten Betätigungselement, wie beispielsweise zwei Griffstücken oder einem Daumenhebel verbunden ist. Durch eine Betätigung der proximalseitigen Betätigungselemente wird die Winkelstellung des distal angeordneten Eingriffselement, das bei dem dargestellten Ausführungsbeispiel ohne Beschränkung des allgemeinen Erfindungsgedankens eine Schere ist, relativ zu dem als Einheit schwenkbaren Element geändert.

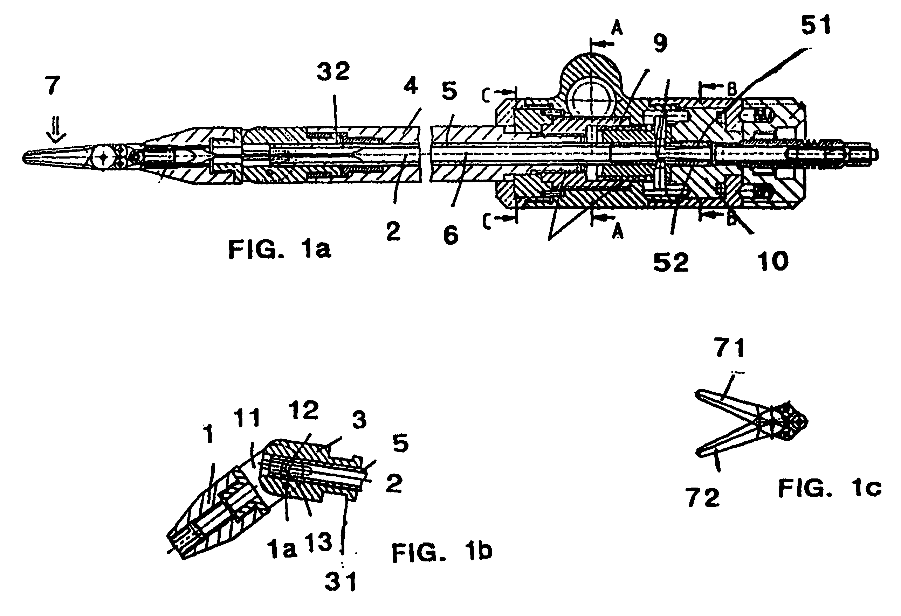

Figur 1c zeigt eine Möglichkeit für die Betätigung der Scherenmäuler 71 und 72.

Da die Schwenkachse 1a des als Einheit schwenkbaren bzw. abwinkelbaren distalen Endes 1 die Längsachse 2 des endoskopischen Instruments nicht schneidet, würde sich die Winkelstellung des distal vorgesehenen Eingriffselements 7 relativ zu dem Element 1 bei einer Schwenkung ändern.

Deshalb ist erfindungsgemäß ein Ausgleichsmechanismus vorgesehen, der diese Änderung verhindert und der im folgenden erläutert werden wird.

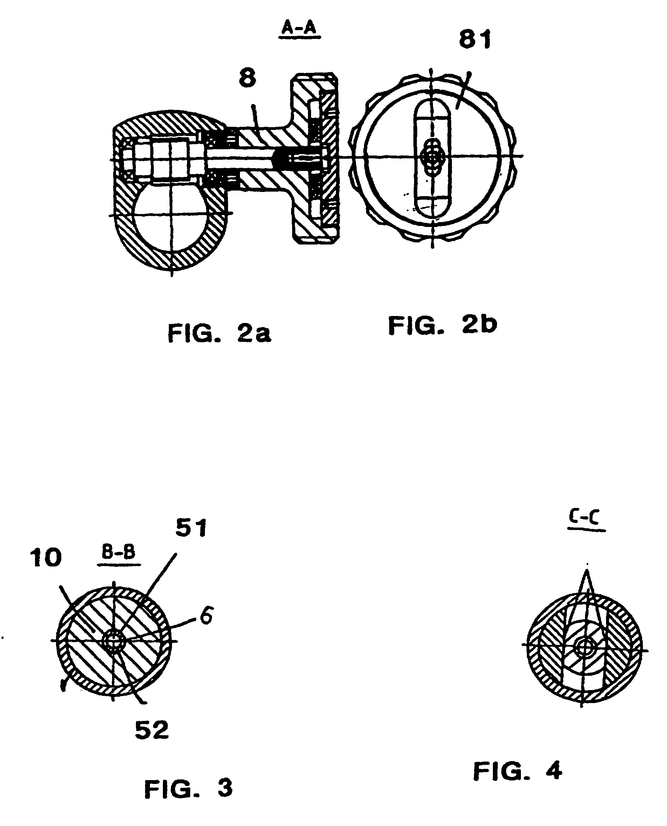

Zur Einstellung der Winkellage des Elements 1 ist ein als Stellschraube 8 ausgebildete Stellelement vorgesehen (Fig. 2a). Figur 2b zeigt eine Aufsicht auf dem Betätigungsknopf 81 der Stellschraube 8. Die Stellschraube 8 steht über einem Gewindeeingriff mit einem Zwischenelement 9 in Verbindung. Das Zwischenelement 9 dreht über ein Linksgewinde den Hohlzylinder 5 und verschiebt über ein Rechtsgewinde das Außenrohr 4. Die Gewindesteigungen sind so bemessen, daß ein Längenausgleich bei einer Abwinkelung des Elements 1 erfolgt. Zur Betätigung des Hohlzylinders bzw. des Außenrohrs sind Zapfen vorgesehen, die an entsprechenden Anlageflächen anliegen. Zu einer möglichen konstruktiven Ausgestaltung wird auf Fig. 1a verwiesen.

Zur Verdrehsicherung weist der Hohlzylinder 5 an seinem proximalen Ende zwei sich gegenüberliegende Erhöhungen 51 und 52 auf, die gefedert sind. Mit diesen Erhöhungen 51 und 52 ist er in komplementär ausgebildeten Aufnahmen eines Rohres 10 einsetzbar. Wenn die Stange 2 in das Instrument eingesetzt ist, ist ein Einfedern der Erhöhungen 51 und 52 nicht mehr möglich (vgl. auch Figur 3), so daß eine verdrehung des Hohlzylinders nicht mehr möglich ist.

Figur 4 stellt die Verdrehsicherung des Gewindeantriebs am Außenrohr dar.

## Patentansprüche

1. Endoskopisches Instrument mit
- proximal angeordneten Betätigungselementen,
- einem Außenrohr (4),
- einem an dem distalen Ende des Außenrohrs (4) um eine Schwenkachse (1a) mittels einer Stellschraube (8) schwenkbar angelenkten Element (1),
- einem Stellelement (8), durch dessen Betätigung das Element (1) schwenkbar ist,
- einem an dem schwenkbaren Element (1) distal angeordneten Eingriffselement (7;71;72), das über eine Stange (6) mit den proximal angeordneten Betätigungselementen derart verbunden ist, dass die Winkelstellung des Eingriffselements (7;71;72) relativ zu dem schwenkbaren Element (1) geändert werden kann,
wobei
- die Stange (6) in einem in dem Außenrohr (4) geführten Hohlzylinder (5) geführt ist,
- die Schwenkachse (1a) von der Mittelachse (2) des Hohlzylinders (5) in Richtung senkrecht zur Mittelachse (2) einen Abstand aufweist, wobei die Stellschraube (8) über einen Gewindeeingriff den Hohlzylinder (5) verschiebt, der konzentrisch zu der Stange (6) angeordnet ist, und der ein Schwenkelement betätigt, das die Winkelstellung des Elements (1) ändert und
- das Instrument ferner einen Ausgleichmechanismus aufweist, der eine Änderung der durch die Betätigung der Betätigungselemente eingestellten Winkelstellung des Eingriffselements (7;71;72) relativ zu dem schwenkbaren Element (1) bei einer Schwenkung dieses Elements (1) um die Schwenkachse (1a) verhindert,
wobei
- der Ausgleichsmechanismus darin besteht, dass die Stellschraube (8) über einen Gewindeeingriff ein Zwischenelement (9) dreht, das den Hohlzylinder (5) über ein Linksgewinde verschiebt und das Außenrohr (4) über ein Rechtsgewinde verschiebt.

2. Instrument nach Anspruch 1,
**dadurch gekennzeichnet, dass** die Stange (6) im Bereich der Abwinkelung flexibel ausgebildet ist.

3. Instrument nach Anspruch 2,
**dadurch gekennzeichnet, dass** die Stange (6) im Bereich der Abwinkelung einen geringeren Durchmesser als in dem nicht abgewinkelten Bereich aufweist.

4. Instrument nach einem der Ansprüche 1 bis 3,
**dadurch gekennzeichnet, dass** das Eingriffselement (7) eine Schere oder eine Zange ist.

5. Instrument nach Anspruch 1,
**dadurch gekennzeichnet, dass** das Schwenkelement (11) und der Hohlzylinder (5) über einen Zapfen (13), der in eine Nut (12) eingreift, miteinander in Wirkverbindung stehen.

6. Instrument nach Anspruch 1,
**dadurch gekennzeichnet, dass** das Schwenkelement (11) mit dem distalen Ende (1) fest verbunden ist.

7. Instrument nach einem der Ansprüche 1 bis 6,
**dadurch gekennzeichnet, dass** die Stellschraube (8) unter einem Winkel von 90° zur Längsache (2) des Instruments angeordnet ist.

8. Instrument nach einem der Ansprüche 1 bis 6,
**dadurch gekennzeichnet, dass** die Stellschraube (8) konzentrisch zur Längsache (2) des Instruments angeordnet ist.

9. Instrument nach einem der Ansprüche 1 bis 8,
**dadurch gekennzeichnet, dass** bestimmte Winkelstellungen des distalen Endes (1) indexiert sind, oder dass die Stellschraube (8) eine Rastung bei bestimmten Winkelstellungen aufweist.

10. Instrument nach einem der Ansprüche 1 bis 9,
**dadurch gekennzeichnet, dass** der Hohlzylinder (5) der das distale Ende (1) als Einheit schwenkt, distalseitig in einem Bajonettelement (3) geführt ist, das einen Bajonettflansch (31) aufweist, mit dem es in eine Bajonettaufnahme (32) in einem Außenrohr einsetzbar ist.

11. Instrument nach Anspruch 10,
**dadurch gekennzeichnet, dass** der Hohlzylinder (5) an seinem proximalen Endbereich zwei sich gegenüberliegende gefederte Erhöhungen (51, 52) aufweist, mit denen er in komplementär ausgebildete Aufnahmen eines verdrehgesicherten Rohrs (10) einsetzbar ist.

12. Instrument nach Anspruch 10 oder 11,
**dadurch gekennzeichnet, dass** die Sicherung des Hohlzylinders (5) gegen Verdrehen und damit ein Öffnen des Bajonetts über die eingesetzte Stange (6) erfolgt, die ein Einfedern der Erhöhungen (51, 52) des Hohlzylinders (5) verhindert.

## Claims

1. Endoscopic instrument, comprising:
- proximally disposed actuator elements;
- an outer tube (4);
- an element (1) pivotally supported on the distal end of the outer tube (4) and adapted to be swivelled by means of an adjusting screw (8) around a swivel axis (1a);
- an adjusting element (8), actuation of which swivels the element (1);
- an operating element (7; 71; 72) distally disposed on the swivelling element (1), and connected via a rod (6) to the proximally disposed actuator elements in such manner that the angular position of the operating element (7; 71; 72) relative to the swivelling element (1) can be changed;
wherein
- the rod (6) is guided in a hollow cylinder (5) that is guided in the outer tube (4);
- the swivel axis (1a) is spaced from the centre axis (2) of the hollow cylinder (5) in a direction perpendicular to the centre axis (2), wherein the adjusting screw (8) slides the hollow cylinder (5) by means of a screw-thread engagement, the hollow cylinder being disposed to be concentric with the rod (6) and actuating a swivel element which changes the angular position of the element (1); and
- the instrument is furthermore provided with a compensating mechanism for preventing a change of the angular position, set by an actuation of the actuator elements, of the operating element (7; 71; 72) relative to the swivelling element (1) during a swivelling movement of the element (1) about the swivel axis (1a);
wherein
- the compensating mechanism consists of the adjusting screw (8) rotating an intermediate element (9) via a screw-thread engagement, said intermediate element sliding the hollow cylinder (5) via a lefthand thread, and sliding the outer tube (4) via a right-hand thread.

2. Instrument according to claim 1,
**characterized in that** in the angled portion thereof the rod (6) is designed to be flexible.

3. Instrument according to claim 2,
**characterized in that** in the angled portion thereof the rod (6) has a smaller diameter than in the non-angled portion.

4. Instrument according to any one of claims 1 to 3,
**characterized in that** the operating element (7) consists of cutters or forceps.

5. Instrument according to claim 1,
**characterized in that** the swivel element (11) and the hollow cylinder (5) are interactively connected via a pin (13) engaging in a groove (12).

6. Instrument according to claim 1,
**characterized in that** the swivel element (11) is firmly connected to the distal end (1).

7. Instrument according to any one of claims 1 to 6,
**characterized in that** the adjusting screw (8) is disposed at an angle of 90° to the longitudinal axis (2) of the instrument.

8. Instrument according to any one of claims 1 to 6,
**characterized in that** the adjusting screw (8) is disposed to be concentric with the longitudinal axis of the instrument.

9. Instrument according to any one of claims 1 to 8,
**characterized in that** particular angular positions of the distal end (1) are indexed, or that the adjusting screw (8) locks-in at particular angular positions.

10. Instrument according to any one of claims 1 to 9,
**characterized in that** the hollow cylinder (5) which swivels the distal end (1) as a unit is guided on the distal side in a bayonet element (3) having a bayonet flange (31) with which said bayonet element is adapted to be inserted into a bayonet socket (32) in the outer tube.

11. Instrument according to claim 10,
**characterized in that** the hollow cylinder (5) is provided at its proximal end portion with two oppositely disposed spring-loaded elevations (51, 52) with which said cylinder is adapted to be inserted into complementarily designed seats of a tube (10) that is secured against rotational movement.

12. Instrument according to claim 10 or 11,
**characterized in that** a securing of the hollow cylinder (5) against rotational movement and thus an opening of the bayonet occurs via the inserted rod (6) which prevents a locking-in of the elevations (51, 52) of the hollow cylinder (5).

## Revendications

1. Instrument endoscopique comprenant
- des éléments de commande, disposés du côté proximal,
- un tube extérieur (4),
- un élément (1) articulé à pivoter, moyennant un vis de réglage (8), à une extrémité distale dudit tube extérieur (4),
- un élément de réglage (8) dont la commande permet le pivotement dudit élément (1),
- un élément de prise (7 ; 71 ; 72) disposé, du côté distal, audit élément pivotable (1), qui est relié via une tige (6) aux éléments de commande disposés du côté proximal, d'une telle manière, que la position angulaire dudit élément de prise (7 ; 71 ; 72) soit variable relativement audit élément pivotable (1),
dans lequel
- ladite tige (6) est guidée au-dedans d'un cylindre creux guidé dans ledit tube extérieur (4),
- l'axe de pivotement (1 a) présentant une distance à partir de l'axe central (2) dudit cylindre creux (2) en un sens orthogonal sur l'axe central (2), à ladite vis de réglage (8) déplaçant, via une prise par filetage, ledit cylindre creux (5), qui est disposé en position concentrique relativement à ladite tige (6), et qui commande un élément pivotant, qui varie la position angulaire dudit élément (1), et
- dans lequel l'instrument comprend au plus un mécanisme compensateur, qui empêche une variation de la position angulaire dudit élément de prise (7 ; 71 ; 72), qui est ajustée par la commande des éléments de commande, relativement audit élément pivotable (1) en cas d'un pivotement de cet élément (1) autour de l'axe de pivotement,
dans lequel
- ledit mécanisme compensateur consiste en la mesure que ladite vis de réglage (8), via une prise par filetage, tourne un élément intermédiaire, qui déplace ledit cylindre creux (5) via un filet à gauche et déplace ledit tube extérieur (4) via un filet à droite.

2. Instrument selon la revendication 1,
**caractérisé en ce que** ladite tige (6) a une configuration flexible dans la zone de son pliage.

3. Instrument selon la revendication 2,
**caractérisé en ce que** ladite tige (6) a un diamètre dans la zone de pliage, qui est plus petit que le diamètre dans la zone non pliée.

4. Instrument selon une quelconque des revendications 1 à 3,
**caractérisé en ce que** ledit élément de prise (7) est une cisaille ou une pince.

5. Instrument selon la revendication 1,
**caractérisé en ce que** ledit élément pivotant (11) et ledit cylindre creux (5) se trouvent en connexion effective active l'un avec l'autre via un pivot (13) en prise dans une rainure (12).

6. Instrument selon la revendication 1,
**caractérisé en ce que** ledit élément pivotant (11) est relié, de façon fixe, à l'extrémité distale (1).

7. Instrument selon une quelconque des revendications 1 à 6,
**caractérisé en ce que** ladite vis de réglage (8) est disposée à un angle de 90° relatif à l'axe longitudinal (2) de l'instrument.

8. Instrument selon une quelconque des revendications 1 à 6,
**caractérisé en ce que** ladite vise de réglage (8) se trouve en arrangement concentrique relatif à l'axe longitudinal (2) de l'instrument.

9. Instrument selon une quelconque des revendications 1 à 8,
**caractérisé en ce que** certains positions définies de l'extrémité distale (1) sont indexées, ou **en ce que** ladite vis de réglage (8) présente un effet à crans aux positions angulaires définies.

10. Instrument selon une quelconque des revendications 1 à 9,
**caractérisé en ce que** ledit cylindre creux (5), qui pivote l'extrémité distale (1) comme une unité, est guidé, du côte distal, à l'intérieur d'un élément à baïonnette (3), qui comprend un collet à baïonnette (31) par lequel il se peut introduire dans un logement (32) à baïonnette (32) dans un tube extérieur.

11. Instrument selon la revendication 10,
**caractérisé en ce que** ledit cylindre creux (5) comprend deux bosses à ressort opposées (51, 62), moyennant lesquelles il se peut introduire dans des logements à configuration complémentaire d'un tube (10) à protection antitorsion (10).

12. Instrument selon la revendication 10 ou 11,
**caractérisé en ce que** la protection antitorsion dudit cylindre creux (5) et ainsi une ouverture du baïonnette se font via ladite tige (6) insérée, qui empêche une compression desdites bosses (51, 52) dudit cylindre creux (5).
